# EUROPÄISCHE PATENTANMELDUNG

(11) **EP 0 549 872 A2**
(43) Veröffentlichungstag der Anmeldung: **07.07.1993**
(21) Anmeldenummer: 92119212.6
(22) Anmeldetag: 10.11.1992
(51) Int. Cl.: C12P 41/00, C12P 7/04, C07C 69/007, C07C 69/63

(54) **Verfahren zur Herstellung von (2R,3E)-4-Halo-3-buten-2-olen**

(30) Priorität: 15.11.1991 CH 3339/91
(71) Anmelder: LONZA AG, CH-4002 Basel (CH)
(72) Erfinder: Bänziger, Markus, Dr., Brig-Glis (Kanton Wallis) (CH); McGarrity, John, Dr., Visp (Kanton Wallis) (CH)
(74) Vertreter: Weinhold, Peter, Dr.

(57) **Zusammenfassung**

(2R,3E)-4-Halo-3-buten-2-ole werden durch kinetische Racematspaltung aus dem entsprechenden Racematen hergestellt. Das Racemat wird durch Umsetzung mit einem Carbonsäurederivat, vorzugsweise mit Chloracetylchlorid, verestert. Anschliessend wird mit einer Lipase aus Pseudomonas fluorescens der (R)-Ester enantioselektiv hydrolysiert. Aus dem zurückbleibenden (S)-Ester kann nach Abtrennung auch das entsprechende (2S,3E)-4-Halo-3-buten-2-ol gewonnen werden. Die Verbindungen sind chirale Synthesebausteine für die Herstellung von optisch aktiven Naturstoffen.

## Beschreibung

Gegenstand der vorliegenden Erfindung ist ein Verfahren zur Herstellung von (2R,3E)-4-Halo-3-buten-2-olen aus den entsprechenden racemischen (2RS,3E)-4-Halo-3-buten-2-olen durch Veresterung und enantioselektive Hydrolyse der Ester.

Optisch aktive sekundäre γ-Haloallylalkohole, insbesondere die γ-Iodallylalkohole, sind wertvolle Synthesebausteine für die Herstellung von Prostaglandinen (s. z.B. F.-T. Luo und E.-I. Negishi, J. Org. Chem. **1985**, 50, 4762-4766) oder von optisch aktiven Propargylalkoholen, die ihrerseits Bausteine für verschiedene Naturstoffe darstellen (s. z.B. T. Ito et al., Tetrahedron Sett. **1989**, 30, 7083-7086).

Da racemische γ-Haloallylalkohole leicht aus Carbonsäurechloriden und Acetylen über die entsprechenden Chlorvinylketone erhältlich sind (s. z.B. Y. Kitano et al., Tetrahedron Lett. **1987**, 30, 6351-6354), werden die optisch aktiven Verbindungen zweckmässig durch Racematspaltung hergestellt. Ein bekanntes Verfahren hierzu ist die kinetische Racematspaltung durch Epoxidierung nach Sharpless in Gegenwart einer chiralen Hilfssubstanz (Y. Kitano et al., loc. cit.). Nachteile dieses Verfahrens sind die Verwendung von Peroxyverbindungen, die in grösseren Mengen ein Sicherheitsrisiko darstellen, sowie die Tatsache, dass ein Enantiomer verlorengeht, indem sich das daraus gebildete Epoxid bei der Aufarbeitung zersetzt.

Aufgabe der vorliegenden Erfindung war die Bereitstellung eines Verfahrens zur Herstellung von (2R,3E)-4-Halo-3-buten-2-olen, das ohne Verwendung von Peroxyverbindungen auskommt und es ermöglicht, auch das (2S)-Enantiomere zu verwerten.

Erfindungsgemäss wird die Aufgabe durch das Verfahren nach Patentanspruch 1 gelöst.

Es wurde gefunden, dass sich die aus racemischen (E)-4-Halo-3-buten-2-olen nach bekannten Methoden herstellbaren Ester der allgemeinen Formel 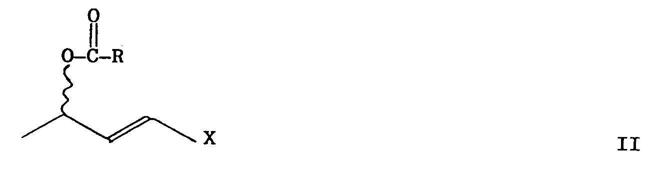
worin R eine gegebenenfalls mit Halogen substituierte Alkylgruppe mit 1 bis 8 Kohlenstoffatomen und X Chlor, Brom oder Iod ist, mit einer Lipase aus Pseudomonas fluorescens enantioselektiv hydrolysieren lassen. Der nicht hydrolysierte (2S,3E)-Ester kann aufgrund seiner unterschiedlichen physikalischen Eigenschaften leicht von dem Hydrolyseprodukt (2R,3E)--4-Halo-3-buten-2-ol abgetrennt und anschliessend zu (2S,3E)-4-Halo-3-buten-2-ol hydrolysiert werden.
Bevorzugte Ester sind die der Carbonsäuren mit 2 bis 8 Kohlenstoffatomen und ihrer halogenierten Derivate, besonders bevorzugt sind die Ester der Chloressigsäure (R = Chlormethyl). Die Ester werden vorzugsweise durch Umsetzung des racemischen (E)-4-Halo-3-buten-2-ols mit dem entsprechenden Säureanhydrid oder Säurechlorid, also beispielsweise für den Chloressigsäu-reester mit Chloracetylchlorid, in Gegenwart eines tertiären Amins, wie beispielsweise Triethylamin, zur Bindung des entstehenden Chlorwasserstoffs hergestellt. Vorteilhaft wird dabei noch ein Katalysator, wie beispielsweise 4-(Dimethylamino)pyridin, verwendet.

Die enantioselektive Hydrolyse des Esters wird erfindungsgemäss mit einer Lipase aus Pseudomonas fluorescens durchgeführt. Solche Lipasen sind im Handel, beispielsweise von der Firma Amano oder der Firma Biocatalysts, erhältlich.
Vorzugsweise wird die enantioselektive Hydrolyse in einem gerührten zweiphasigen System aus Wasser und einem nicht mit Wasser mischbaren organischen Lösungsmittel durchgeführt. Besonders bevorzugt als organisches Lösungsmittel ist Toluol. Die Reaktionstemperatur beträgt zweckmässig 0 bis 50°C, vorzugsweise 5 bis 25°C.

Um unerwünschte Nebenreaktionen auszuschliessen, wird die Hydrolyse zweckmässig bei einem pH von 5 bis 9 durchgeführt, vorzugsweise bei einem pH von 6 bis 8.
Da bei der Hydrolyse Säure frei wird, wird der pH zweckmässig durch Pufferzusatz oder vorzugsweise durch eine entsprechend dem Reaktionsfortschritt erfolgende Zugabe einer Base konstant gehalten. Die Basenzugabe wird vorzugsweise über ein pH-Messgerät und ein automatisches Dosiergerät (Autotitrator) vorgenommen. Als Base wird vorzugsweise eine wässrige Alkalihydroxidlösung eingesetzt, besonders bevorzugt ist Natronlauge.

Nachdem die Hauptmenge des (R)-Esters hydrolysiert ist, also nach einem Umsatz von annähernd 50%, bezogen auf das Racemat, wird die Reaktion abgebrochen und der optisch aktive Alkohol von dem unumgesetzten Ester abgetrennt. Die Trennung wird vorteilhaft durch fraktionierende Destillation unter vermindertem Druck durchgeführt.

Die nachfolgenden Beispiele verdeutlichen die Durchführung des erfindungsgemässen Verfahrens.

### Beispiel 1

### (E)-4-Iod-3-buten-2-on

In 125 ml Aceton wurden 50 g (0,48 mol) (E)-4-Chlor-3-buten-2-on (hergestellt nach W. B. Benson, J. Org. Chem. **1964**, 29, 385) gelöst und unter Argon mit 89,4 g (0,60 mol) Natriumiodid 1,8 h bei 60°C gerührt. Anschliessend wurde das Reaktionsgemisch bei 130 mbar und 35°C Badtemperatur im Rotationsverdampfer eingeengt. Der Rückstand wurde mit 150 ml Toluol versetzt und die so erhaltene Suspension mit 150 ml Wasser gewaschen. Die Wasserphase wurde dreimal mit je 100 ml Toluol extrahiert. Die vereinigten organischen Phasen wurden über Magnesiumsulfat getrocknet und am Rotationsverdampfer eingeengt.
- Ausbeute:: 85 g (Rohprodukt)

### Beispiel 2

### (2RS,3E)-4-Iod-3-buten-2-ol

Unter Argon wurden 85 g (0,43 mol) (E)-4-Iod-3-buten-2-on (hergestellt nach Beispiel 1) in 800 mol Toluol gelöst. Die Lösung wurde auf -10°C abgekühlt und innerhalb von 0,5 h tropfenweise mit 65 ml einer 3,5 M Lösung von Natriumdihydridobis(2-methoxyethoxy)aluminat in Toluol versetzt. Anschliessend wurde das Gemisch zunächst auf Raumtemperatur erwärmt, dann auf 0°C abgekühlt und bei dieser Temperatur vorsichtig zuerst mit 10 ml Methanol und dann mit 40 ml 10%-iger Natronlauge versetzt. Die Phasen wurden getrennt und die Wasserphase mit 200 ml Toluol extrahiert.Die Toluolphase wurde einmal mit 200 ml und zweimal mit 100 ml Wasser gewaschen, anschliessend über Magnesiumsulfat getrocknet und am Rotationsverdampfer eingeengt.
- Ausbeute:: 67,7 g (Rohprodukt, Gehalt (GC) 85%)
- ¹H NMR: (CDCl₃, 300 MHz) δ: 1,3 (d, 3H); 2,05 (br.d, 1H); 4,23-4,36 (m, 1H); 6,35 (d, 1H); 6,62 (dd, 1H).

### Beispiel 3

### Chloressigsäure-(2RS,3E)-4-iod-3-buten-2-ylester

Unter Argon wurden 57,1 g (0,25 mol) (2RS,3E)-4-Iod-3-buten-2-ol (86%-ig, hergestellt nach Beispiel 2) in 400 ml Toluol gelöst, auf 0°C abgekühlt und mit 2,4 g (20 mmol) 4-(Dimethylamino)pyridin versetzt. Anschliessend wurde das Gemisch auf -5°C abgekühlt, mit 11,07 g Triethylamin versetzt und weiter auf -10°C abgekühlt.
Nach tropfenweiser Zugabe einer ersten Portion von 12,4 g Chloracetylchlorid innerhalb von 15 min (exotherme Reaktion) und 0,5 h Nachrühren wurden nacheinander nochmals die gleiche Menge Triethylamin und Chloracetylchlorid und nach weiteren 0,5 h schliesslich jeweils eine dritte Portion zugetropft, insgesamt also 33,21 g Triethylamin und 37,2 g Chloracetylchlorid. Während der Zugabe des Chloracetylchlorids fiel Triethylammoniumchlorid aus. Nach der letzten Zugabe wurde das Gemisch noch 1,5 h bei -5°C gerührt und dann auf Raumtemperatur erwärmt. Nach weiteren 3 h wurde das Triethylammoniumchlorid abfiltriert und mit Toluol gewaschen. Das Filtrat wurde dreimal mit je 80 ml 0,1 M Salzsäure gewaschen. Die Wasserphase wurde einmal mit 50 ml Toluol zurückextrahiert. Die vereinigten organischen Phasen wurden über Magnesiumsulfat getrocknet, filtriert und am Rotationsverdampfer eingeengt.
- Ausbeute:: 72,4 g (Rohprodukt, enthält noch etwas Toluol)
- ¹H-NMR: (CDCl₃, 300 MHz) δ: 1,38 (d, 3H); 4,05 (s, 2H); 5,30-5,43 (m, 1H); 6,50-6,58 (m, 2H).

### Beispiel 4

### (2R,3E)-4-Iod-3-buten-2-ol

In 136 ml Toluol wurden 72 g Chloressigsäure-(2RS,3E)-4-iod-3-buten-2-ylester (hergestellt nach Beispiel 3) gelöst. Die Lösung wurde bei Raumtemperatur mit 680 ml Wasser und 680 mg Lipase PS (aus Pseudomonas fluorescens, Hersteller Amano) gerührt, wobei der pH mit einer Glaselektrode gemessen und durch Zugabe von 1 M Natronlauge mittels eines Autotitrators konstant bei 7 gehalten wurde. Nach 2,5 h wurde die Reaktion abgebrochen. Das Reaktionsgemisch wurde über Celite^{R} abfiltriert, mit weiteren 160 ml Toluol versetzt und geschüttelt. Nach Trennung der Phasen wurde die Wasserphase noch zweimal mit je 160 ml Toluol extrahiert. Die vereinigten organischen Phasen wurden über Magnesiumsulfat getrocknet, filtriert und am Rotationsverdampfer eingeengt.
Das so erhaltene Rohprodukt (57,4 g) wurde über eine Spaltrohrkolonne rektifiziert.
Ausbeute: 16,65 g (entspr. 34%, bezogen auf racemisches (E)-4-Iod-3-buten-2-ol, oder 68% d.Th)
Gehalt (GC) ≃100%
Optische Reinheit (ee-Wert): >99%

## Patentansprüche

1. Verfahren zur Herstellung von (2R,3E)-4-Halo-3-buten-2-olen der allgemeinen Formel 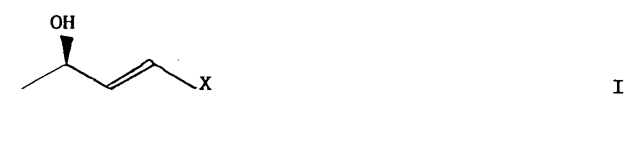 worin X Chlor, Brom oder Iod ist, aus den entsprechenden racemischen (2RS,3E)-4-Halo-3-buten-2-olen, dadurch gekennzeichnet, dass der racemische Alkohol in einen entsprechenden racemischen Ester 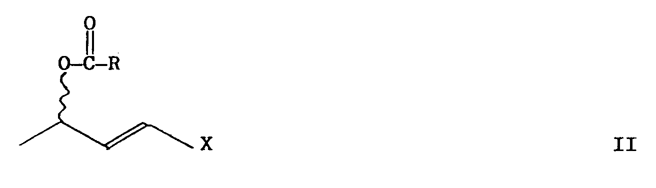 worin R eine gegebenenfalls mit Halogen substituierte Alkylgruppe mit 1 bis 8 Kohlenstoffatomen ist und X die oben genannte Bedeutung hat, übergeführt und anschliessend mit einer Lipase aus Pseudomonas fluorescens enantioselektiv hydrolysiert wird.

2. Verfahren nach Patentanspruch 1, dadurch gekennzeichnet, dass R Chlormethyl ist.

3. Verfahren nach Patentanspruch 2, dadurch gekennzeichnet, dass der racemische Ester durch Umsetzung des racemischen Alkohols mit Chloracetylchlorid in Gegenwart eine tertiären Amins hergestellt wird.

4. Verfahren nach einem oder mehreren der Patentansprüche 1 bis 3, dadurch gekennzeichnet, dass die enantioselektive Hydrolyse in einem zweiphasigen System aus Wasser und einem mit Wasser nicht mischbaren organischen Lösungsmittel durchgeführt wird.

5. Verfahren nach einem oder mehreren der Patentansprüche 1 bis 4, dadurch gekennzeichnet, dass die enantioselektive Hydrolyse bei einem pH von 6 bis 8 durchgeführt wird.

6. Verfahren nach einem oder mehreren der Patentansprüche 1 bis 5, dadurch gekennzeichnet, dass X Iod ist.
